# EUROPEAN PATENT APPLICATION

(11) **EP 1 433 784 A1**
(43) Date of publication of application: **30.06.2004**
(21) Application number: 03029586.9
(22) Date of filing: 22.12.2003
(51) Int. Cl.: C07D 213/74

(54) **Process for the synthesis of torsemide, in particular of pure and stable form II**

(30) Priority: 23.12.2002 IT MI20022749
(71) Applicant: Cosma S.p.A., 24040 Ciserano (IT)
(72) Inventor: Lusanna, Massimiliano, 24020 Gorle (IT); Rainoni, Mauro, 20063 Cernusco Sul Naviglio (IT); Gambuzza, Filippo, 24030 Terno D'Isola (IT)
(74) Representative: Gervasi, Gemma, Dr.

(57) **Abstract**

The present invention relates to a new process for the synthesis of torsemide, in particular of pure and stable form II, which comprises direct synthesis of torsemide from 4-(3-methylphenylamino)-3-pyridine-sulphonamide. The new process envisages fewer steps than the processes described in the prior art, with improved yields and good quality from the chemical and preferably polymorphous points of view.

## Description

### FIELD OF THE INVENTION

The present invention relates to a new process for the synthesis of torsemide, in particular of its form II pure and stable, the said process comprising direct synthesis of torsemide from 4-(3-methylphenylamino)-3-pyridine-sulphonamide. The new process envisages fewer steps than the processes described in the prior art, with improved yields and good quality from the chemical and preferably polymorphous points of view.

### PRIOR ART

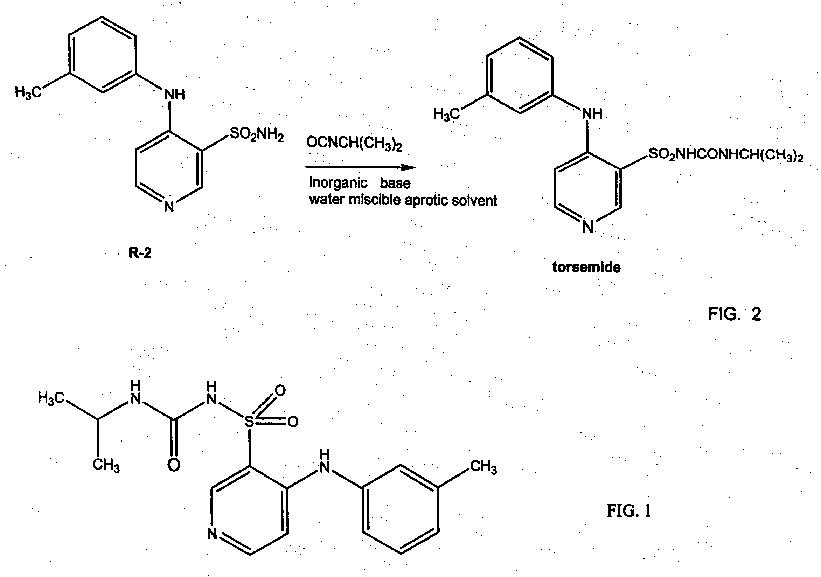

Torsemide (or torasemide), i.e., 1-isopropyl-3-[(4-m-toluidino-3-pyridyl)sulphonyl]-urea or else *N*-[[(1-methylethyl)amino]carbonyl]-4-[(3-methylphenyl)amino]-3-pyridinesulphonamide, or else 3-isopropylcarbamylsulphonamido-4-(3'-methylphenyl)aminopyridine, i.e., C₁₆H₂₀N₄O₃S, molecular weight 348,43, CAS No. 56211-40-6, described for the first time in the example 71 of the West German patent DE 25 16 025 which dates back to 1974, is a compound having a well-known applicability in the pharmaceutical field, in particular, for the fabrication of diuretic agents. Recently, torsemide has also been proposed for the treatment of cerebral oedema (US 5,486,530). It is known that torsemide, in the solid state, can exist in a number of crystalline modifications, the so-called polymorphous forms. Various polymorphs of torsemide, as well as further non-crystalline forms, the so-called amorphous ones, the processes for obtaining them and their peculiarities are described in the US patents Re-issue 34,672 and Re-issue 34,580, in "Structure cristalline et moléculaire d'un diurétique dérivé de l'alkyl-1[(phénylamino-4-pyridyl-3)sulfonyl]-3 urée: la torasémide (C₁₆H₂₀N₄O₃S)" by Dupont *et al*. in *Acta Cryst.* (1978) B34, 1304-1310, in "Structure d'une seconde variété de la torasémide" in *Acta Cryst*. (1978) B34, 2659-2662, in "Chemical structure and physico-chemical properties of torasemide" by Kondo *et al*. in lyakuhin Kenkyu, Vol. 25, No. 9 (1994) and in US 5,914,336, in WO 00/20935, in WO 01/10441, in WO 01/70694 and in WO 01/87841. The various polymorphous and amorphous modifications of torsemide described above have been designated in different ways by the various researchers who have discovered and/or studied them, and consequently there does not currently exist, in the field of torsemide, a unique and universally accepted nomenclature. For greater clarity, in the framework of the present invention, by ''form I" of torsemide is meant a polymorphous form of torsemide characterized by the x-ray powder diffractogram of the powders illustrated in Figure 1 of the declaration according to CFR § 1.132 by Dr. Fritz Topfmeier, filed on December 30, 1987, which can be found in the file of US 4,822,807, or else as defined in Kondo *et al*. in *lyakuhin Kenkyu*, 25(9), 734-50 (1994), which also describes the respective IR spectrum. Furthermore, in the framework of the present invention, by "form II" of torsemide is meant torsemide characterized by the x-ray powders diffractogram illustrated in Figure 2 of the declaration according CFR § 1.132 by Dr. Fritz Topfmeier, filed on December 30, 1987, which can be found in the file of US 4,822,807, or else as defined in Kondo *et al*, in *lyakuhin Kenkyu*, 25(9), 734-50 (1994), which also describes the respective IR spectrum.

A possible general strategy for the chemical synthesis of torsemide and its homologues has been described in the example 71 of DE 25 16 025, in particular with reference to the first of the eight processes described, illustrated in example 1A for the analogous compound 3-butylcarbamylsulphonamido-4-(3'-chloro)-phenylaminopyridine. In particular, the last step envisages the addition of n-butylisocyanate to 3-sulphonamido-4-(3'-chloro)-phenyl-aminopyridine in the presence of triethylamine. The reaction is protracted for 10 hours at 85-95°C. Alcohol and 2N of NaOH are then added to the residue thus obtained for solubilizing the reaction product, after which the product is precipitated with acetic acid and water, added in excess. The precipitate thus obtained is in turn re-dissolved by means of a treatment with a solution of sodium bicarbonate in a 3:1 water-alcohol mixture, filtered, and re-acidified to obtain the purified solid.

Re 34,672 refers briefly to the method of synthesis as proposed in DE 25 16 025, and in particular to its use, *mutatis mutandis*, for the synthesis of torsemide. The authors of Re 34,672 disclose that in the preparation of torsemide, the raw product obtained - in analogy with the combination of the examples 1A and 71 of DE 25 16 025 - from the reaction between 4-(3-methylphenylamino)-3-pyridine-sulphonamide and isopropyl isocyanate in the presence of triethylamine, after a first cycle of dissolution/precipitation from an aqueous solution for the removal of the reaction mixture, also requires a further purification by means of re-dissolution of the raw product in an aqueous or aqueous-alcoholic solution of sodium bicarbonate, followed by precipitation with acetic acid or CO₂. According to the authors of Re 34,672, torsemide obtained in this way, from the polymorphic point of view, is form II torsemide, which, however, is not stable over time since it slowly transforms into form I.

Also the synthesis of torsemide described in Kondo *et al*. in *lyakuhin Kenkyu*, 25(9), 734-50 (1994) follows the above strategy according to the literature of the known art and proposes, in particular, addition of 4-(3-methylphenylamino)-3-pyridine-sulphonamide (referred to herein as "R-2") to isopropyl isocyanate in the presence of triethylamine in acetone as solvent, elimination of the acetone, addition of water and precipitation of the "raw" torsemide. The latter is re-dissolved in an aqueous solution of sodium hydroxide and, after treatment with activated carbon, the torsemide is re-precipitated with sulphuric acid, adding "seed crystals" of form I (as per Example I of Re 34,672) in order to obtain the torsemide thus repurified in the desired specific crystalline form.

From what has been said above, it emerges that - irrespective of the crystalline form -, preparation of torsemide by means of the addition of its sulphonamide precursor to isopropyl isocyanate in the presence of triethylamine requires (as already summarized in Re-issue 34,672) a first crystallization to eliminate the residue of the reactive mixture, necessarily followed by a second purifying crystallization, possibly with the use of activated carbon as decolouring agent. Said process, in addition to being laborious, implies a considerable loss of substance. Recent efforts aimed at improving accessibility of torsemide in general have been described in WO 01/70226, whose authors point out the poor yields and the need of a number of purifying steps in the already known processes and, in Example 2, propose the addition of 4-(3-methylphenylamino)-3-pyridine-sulphonamide to isopropyl isocyanate in the presence of triethylamine in acetonitrile as solvent. According to the authors of WO 01/70226, the use of acetonitrile as reaction solvent and subsequently - in a further purification step - as means for the extraction of the product already isolated from the reaction mixture, enables a yield of approximately 81.5% of raw torsemide to be obtained. From what has been said above, it is evident that it is still not possible, in the context of known processes of synthesis, to obtain directly and with very high yields a torsemide which will not involve further steps of purification from reaction by-products before its industrial use.

A purpose of the present invention is hence to make available a new process that can be adopted in the last step of synthesis of torsemide (i.e., in the addition of 4-(3-methylphenylamino)-3-pyridine-sulphonamide to isopropyl isocyanate) which will be less laborious than the processes of the known art and will provide improved yields leading directly to a product that is sufficiently pure from the chemical point of view, i.e., preferably with a HPLC purity ≥ 99,0%, for use as active principle in pharmaceutical products.

A further purpose of the present invention is to provide a new process for the synthesis of a "pure and stable" form II torsemide as this has been described in the Italian patent application MI 2002A 000639 in the name of the present applicant, filed on March 27, 2002, the content of which is entirely incorporated herein as reference. "Pure and stable" form II torsemide is of considerable interest in so far as it has been discovered only very recently that - unlike "raw" form II that can be obtained with the various processes of the known art - it can be used for preparing solid pharmaceutical forms. Also US 2002/0035135 describes the possibility of using a form II deemed of "high purity" for obtaining pharmaceutical forms.

In effect, as already discussed above, form II torsemide obtained according to Re. 34,580, as it has been observed by the authors themselves, has proved susceptible to transformation into form I. The same applies to form II torsemide obtained by Kondo *et al*. in *lyakuhin Kenkyu,* 25(9), 734-50 (1994), who note that the mere suspension of form II of torsemide in water leads to the conversion into form I.

Whilst access to "pure and stable" form II torsemide (meaning, "pure and stable" from the polymorphic point of view, that is, characterized in that it does not contain detectable traces of form I, either in the x-ray diffractogram of the powders, or in the FT-IR spectrum recorded in KBr, this latter technique being far more sensitive), described, precisely, in the Italian patent application MI 2002A 000639 in the name of the present applicant, filed on March 27, 2002, entailed a purification of raw torsemide under particular defined and calibrated conditions, a further purpose of the present invention is to make available a new synthetic pathway, that is unique and quantitative from the point of view of crystalline modification, can be adopted in the last step of the synthesis of torsemide (in the addition of 4-(3-methylphenylamino)-3-pyridine-sulphonamide to isopropyl isocyanate), and will lead directly, without isolation of a "raw" torsemide, to "pure and stable" form II torsemide ("pure and stable" meaning free from traces of form I that can be detected in the x-ray diffractogram of the powders as likewise in the FT-IR spectrum recorded in KBr) for use as active principle in pharmaceutical products.

### SUMMARY

To achieve the purposes described above and yet further purposes that will emerge more clearly in what follows, the present invention makes available a process for direct synthesis of torsemide, preferably in its pure and stable form II, by means of the addition of 4-(3-methylphenylamino)-3-pyridine-sulphonamide to isopropyl isocyanate, comprising the following steps:
- providing a reactive mixture comprising 4-(3-methylphenylamino)-3-pyridine-sulphonamide, an aprotic solvent mixable with water or a mixture of aprotic solvents mixable with water and an inorganic base;
- adding, by dripping, to the reactive mixture, isopropyl isocyanate in a substantially equimolar amount to the 4-(3-methylphenylamino)-3-pyridine-sulphonamide included in the reactive mixture, keeping the temperature between 0°C and 70°C;
- optionally adding water upon completion of the reaction;
- removing, by filtration, any insoluble substances that may have been formed; and
- precipitating the torsemide that has formed by acidification.

### DESCRIPTION OF THE FIGURES

The invention, as described in the context of the present patent application, is further illustrated by means of the annexed figures that are described hereibelow. In the ensuing description of the graphic representations, by "Y" axis is meant the vertical axis of the graph and by "X" axis is meant the horizontal axis of the graph.
Figure 1 shows the chemical formula of torsemide.
Figure 2 shows the chemical synthesis of torsemide according to the present invention.
Figure 3 shows the FT-IR spectrum of torsemide obtained according to Example 1. The transmittance (in %) is given on the Y axis, whilst the wave number (in cm⁻¹) appears on the X axis.
Figure 4 shows the FT-IR spectrum of torsemide obtained according to Example 2. The transmittance (in %) is given on the Y axis, whilst the wave number (in cm⁻¹) appears on the X axis.
Figure 5 shows the FT-IR spectrum of torsemide obtained according to Example 3. The transmittance (in %) is given on the Y axis, whilst the wave number (in cm⁻¹) appears on the X axis.
Figure 6 shows the FT-IR spectrum of torsemide obtained according to Example 4. The transmittance (in %) is given on the Y axis, whilst the wave number (in cm⁻¹) appears on the X axis.
Figure 7 shows the FT-IR spectrum of torsemide obtained according to Example 5. The transmittance (in %) is given on the Y axis; whilst the wave number (in cm⁻¹) appears on the X axis.
Figure 8 shows the FT-IR spectrum of torsemide obtained according to Example 6. The transmittance (in %) is given on the Y axis, whilst the wave number (in cm⁻¹) appears on the X axis.
Figure 9 shows the FT-IR spectrum of torsemide obtained according to Example 7. The transmittance (in %) is given on the Y axis, whilst the wave number (in cm⁻¹) appears on the X axis.
Figure 10 shows the FT-IR spectrum of torsemide obtained according to Example 8. The transmittance (in %) is given on the Y axis, whilst the wave number (in cm⁻¹) appears on the X axis.
Figure 11 shows the FT-IR spectrum of torsemide obtained according to Example 9. The transmittance (in %) is given on the Y axis, whilst the wave number (in cm⁻¹) appears on the X axis.
Figure 12 shows the FT-IR spectrum of torsemide obtained according to Example 10. The transmittance (in %) is given on the Y axis, whilst the wave number (in cm⁻¹) appears on the X axis.
Figure 13 shows the x-ray diffractogram of the powders of torsemide obtained according to Example 2. The intensity is given on the Y axis, whilst the angle of reflection (in degrees 2-theta) appears on the X axis.
Figure 14 shows the x-ray diffractogram of the powders of torsemide obtained according to Example 3. The intensity is given on the Y axis, whilst the angle of reflection (in degrees 2-theta) appears on the X axis.
Figure 15 shows the x-ray diffractogram of the powders of torsemide obtained according to Example 4. The intensity is given on the Y axis, whilst the angle of reflection (in degrees 2-theta) appears on the X axis.
Figure 16 shows the x-ray diffractogram of the powders of torsemide obtained according to Example 5. The intensity is given on the Y axis, whilst the angle of reflection (in degrees 2-theta) appears on the X axis.
Figure 17 shows the x-ray diffractogram of the powders of torsemide obtained according to Example 6. The intensity is given on the Y axis, whilst the angle of reflection (in degrees 2-theta) appears on the X axis.
Figure 18 shows the x-ray diffractogram of the powders of torsemide obtained according to Example 7. The intensity is given on the Y axis, whilst the angle of reflection (in degrees 2-theta) appears on the X axis.

### DETAILED DESCRIPTION OF THE INVENTION

The present invention provides a new process for the synthesis of torsemide by means of addition of 4-(3-methylphenylamino)-3-pyridine-sulphonamide to isopropyl isocyanate, which is characterized by yields of pure torsemide of 90-96% (referred to 4-(3-methylphenylamino)-3-pyridine-sulphonamide). The HClO₄ titre of torsemide obtained with the process of the present invention is greater than 99.0%, and preferably, the HPLC titre is equal to or greater than 99.0%, so as to render the torsemide obtained by means of the process according to the present invention suitable for use as active principle in pharmaceutical products.

The new process of the present invention is less laborious than the processes known to the prior art in so far as, after the step of chemical synthesis, it does not require further purification of the product by means of a second salification/dissolution and subsequent re-precipitation of the product from an acidic solution or else by extraction/crushing of the product in the presence of solvent. Instead - according to a first preferred embodiment - it makes directly available a torsemide which is not raw from the chemical standpoint but can be directly used industrially, with very high yields, that have never been achieved in the prior art, already after mere isolation of the torsemide from the reaction mixture.

According to a particularly preferred but non-exclusive embodiment of the new process of the present invention, it is also possible to obtain, for the first time, directly from the reaction mixture, a form II torsemide which is not raw from the polymorphic point of view but is "pure and stable" as defined in Italian patent application MI 2002A 000639 by the present applicant. In particular, "pure and stable" form II torsemide is characterized by the following signals in the x-ray diffractogram of the powders expressed in degrees two-theta: 9.0 ± 0.1; 9.2 ± 0.1; 10.7 ± 0.1; 15.9 ± 0.1; 18.2 ± 0.1; 18.5 ± 0.1; 18.8 ± 0.1; 20.4 ± 0.1; 22.6 ± 0.1; 23.1 ± 0.1; 23.5 ± 0.1, and by the following peaks in the FT-IR spectrum recorded in KBr: 3354 ± 2 cm⁻¹; 3326 ± 2 cm⁻¹; 3085 ± 2 cm⁻¹; 2964 ± 2 cm⁻¹; 1617 ± 2 cm⁻¹; 1555 ± 2 cm⁻¹; 1510 ± 2 cm⁻¹; 1357 ± 2 cm⁻¹; 1326 ± 2 cm⁻¹; 1277 ± 2 cm⁻¹; 1234 ± 2 cm⁻¹; 1151± 2 cm⁻¹; 900 ± 2 cm⁻¹; 837 ± 2 cm⁻¹. More in particular, "pure and stable" form II torsemide is characterized in that it does not contain any detectable contamination with form I, either in the x-ray diffractogram of the powders (in which the signal at 5.7°two-theta indicating any contamination with form I is absent), or in the - markedly more sensitive - FT-IR spectrum recorded in KBr, in which the absence of the peak at 1697 cm⁻¹ (characteristic of form I) may be noted. The absence of the peak at 1697 cm⁻¹ indicates the absence of any form I contamination greater than or equal to 1 wt%, preferably greater than or equal to 0.5 -0.3 wt%.

According to the process of the present invention, torsemide is obtained from 4-(3-methylphenylamino)-3-pyridine-sulphonamide, which, in turn can be obtained with the methods described in the known art from (3-sulphonamido-4-chloro)pyridine, for example as described in US patent 3,904,636. In particular, the 4-(3-methylphenylamino)-3-pyridine-sulphonamide is made available as a component of a reactive mixture comprising an inorganic base and an aprotic solvent mixable with water (or a mixture of aprotic solvents mixable with water), preferably chosen from the group consisting of: acetone, acetonitrile, diglyme, N,N-dimethylformamide, dimethylsulphoxide, *N*-methyl-2-pyrrolidone, dimethylacetamide, and 1,3-dimethyl-2-imidazolidinone. Preferably, the reactive mixture contains also water as further component. Preferably, the aprotic solvent mixable with water is chosen from the group consisting of: acetone, diglyme, *N,N*-dimethylformamide, dimethylsulphoxide, *N*-methyl-2-pyrrolidone, dimethylacetamide, and 1,3-dimethyl-2-imidazolidinone.

Preferably, the inorganic base is chosen from the group consisting of: potassium hydrate, sodium hydrate, sodium carbonate, and potassium carbonate. More preferably, the inorganic base is chosen from the group consisting of potassium hydrate and potassium carbonate. Still more preferably, the inorganic base is potassium carbonate.

Preferably, in the reactive mixture, 4-(3-methylphenylamino)-3-pyridine-sulphonamide is present at 12 -22 wt%, water is present at 0-50 wt%, the inorganic base is present at 3-14 wt%, and the aprotic solvent mixable with water is present at 40-80 wt%.

The reactive mixture referred to above is brought to a temperature (depending on the aprotic solvent mixable with water used and on the inorganic base used) of between 0°C and 70°C and isopropyl isocyanate (IPIC) is added, under stirring, at a rate such as to enable maintenance of the reaction temperature constant and in an amount which is substantially equimolar (i.e., 1.05-1.30 equivalent, preferably 1.10-1.20 equivalent) with respect to the 4-(3-methylphenylamino)-3-pyridine-sulphonamide present in the reactive mixture.

Upon completion of the reaction, the reaction mixture is brought to room temperature and is diluted, optionally, with water, by adding 0-4 parts by weight, referred to the weight of the reactive mixture, any insoluble substances that may be present are filtered, and the torsemide is precipitated by addition of an acid, preferably acetic acid, up to a pH of 5.5 ± 0.2, at a temperature of between 20°C and 25°C. Torsemide is thus obtained with a yield of between 90% and 96% (referred to 4-(3-methylphenylamino)-3-pyridine-sulphonamide), with a HClO₄ titre higher than 99.0% and preferably with a HPLC purity ≥ 99.0% and, in the case where it has been used, as an aprotic solvent mixable in water, a solvent chosen from the group consisting of acetone, diglyme, *N,N*-dimethylformamide, dimethylsulphoxide, *N*-methyl-2-pyrrolidone, dimethylacetamide and 1,3-dimethyl-2-imidazolidinone, in "pure and stable" form II, devoid of any contamination with form I that might be detected by x-ray diffractogram of the powders or by FT-IR spectrum in KBr.

It has thus been shown how the process of synthesis of torsemide provided by the inventors of the present application enables a simplified access to a torsemide with a very high chemical purity, which can be used for the production of pharmaceutical agents, with yields that have never been achieved using processes according to the known art.

Furthermore, according to a particularly preferred embodiment of the present invention it has been made available, for the first time, a direct access to "pure and stable" form II torsemide, which can be used in the pharmaceutical field on account of its high solubility.

### EXPERIMENTAL PART

### Examples of synthesis

### EXAMPLE 1

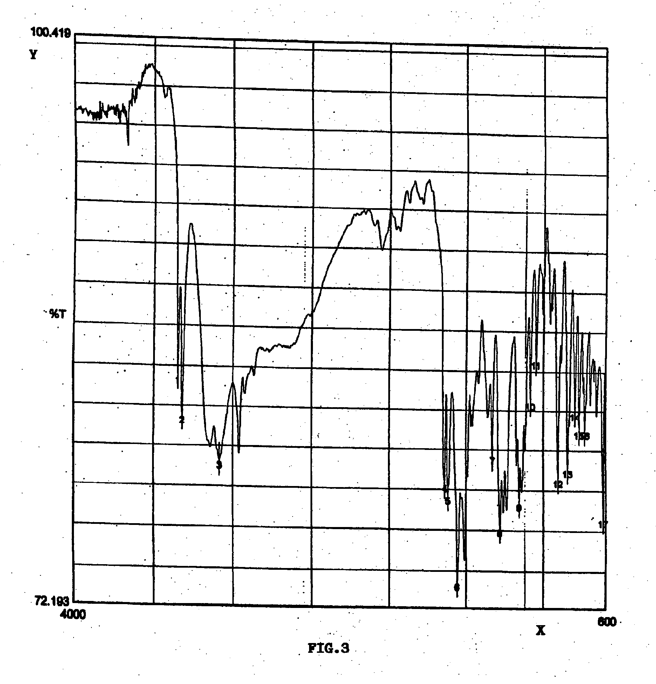

Water (90 cc), potassium hydrate 46.3 wt% (15 g; 0.123 mol) and 4-(3-methylphenylamino)-3-pyridine-sulphonamide (32.5 g; 0.123 mol) are put into a four-necked flask, equipped with mechanical stirrer, thermometer, bubble cooler, and dripping funnel. *N,N*-dimethylformamide (90 cc) is added to the solution thus obtained. The solution is cooled to 0-5°C, and isopropyl isocyanate (12.1 g; 0.142 mol) is added by dripping, keeping the temperature at 0-5°C. At the end of the reaction, the solution is acidified, at 20-25°C, to a pH of 5.5 ± 0.2 with 80% acetic acid (q.s.). The suspension is then stirred for 3 hours at 20-25°C, and is then filtered by suction, thoroughly washing the panel with deionized water. The product is left to dry under vacuum at 60°C, and 38.8 g of torsemide are thus obtained.
(Yield: 90.2% of the theoretical value).
IR: form II (see Figure 3). titre (HClO₄): 99.46 %

### EXAMPLE 2

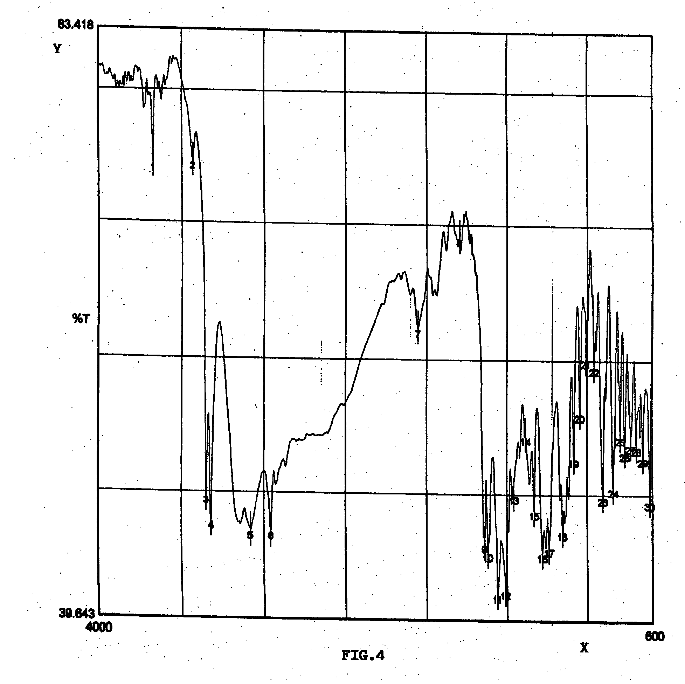

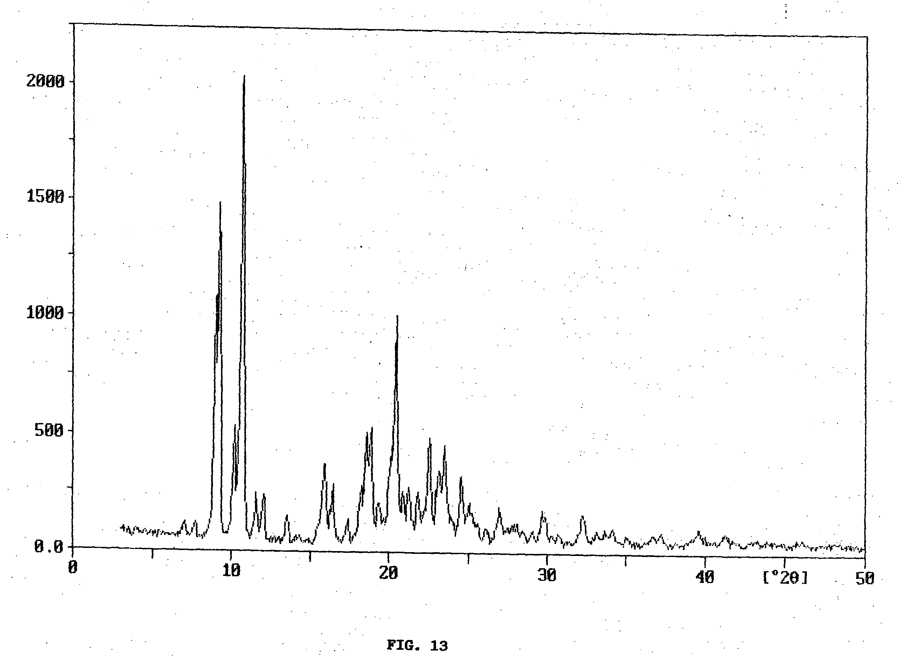

4-(3-methylphenylamino)-3-pyridine-sulphonamide (50 g; 0,189 mol), diglyme (200 cc), potassium carbonate (30 g; 0.217 mol) and water (10 cc) are put into a four-necked flask, equipped with mechanical stirrer, thermometer, bubble cooler, and dripping funnel. The solution is brought to 65-70°C for 15 min and, maintaining said temperature, isopropyl isocyanate (17.7 g; 0.208 mol) is added by dripping for 20 min. At the end of the reaction, water (480 cc) is added, the mixture is filtered by gravity, and is then acidified, at 20-25°C, to a pH of 5.5 ± 0.2 with 80% acetic acid (q.s.). The suspension is then stirred for 3 hours at 20-25°C, and is then filtered by suction, thoroughly washing the panel with deionized water. The product is left to dry overnight at 50°C under vacuum, and 63.86 g of torsemide are thus obtained.
(Yield: 96.5% of the theoretical value).
IR: form II (see Figure 4). DRX: form II (see Figure 13). titre (HClO₄): 99.76% titre (HPLC): 99.18%

### EXAMPLE 3

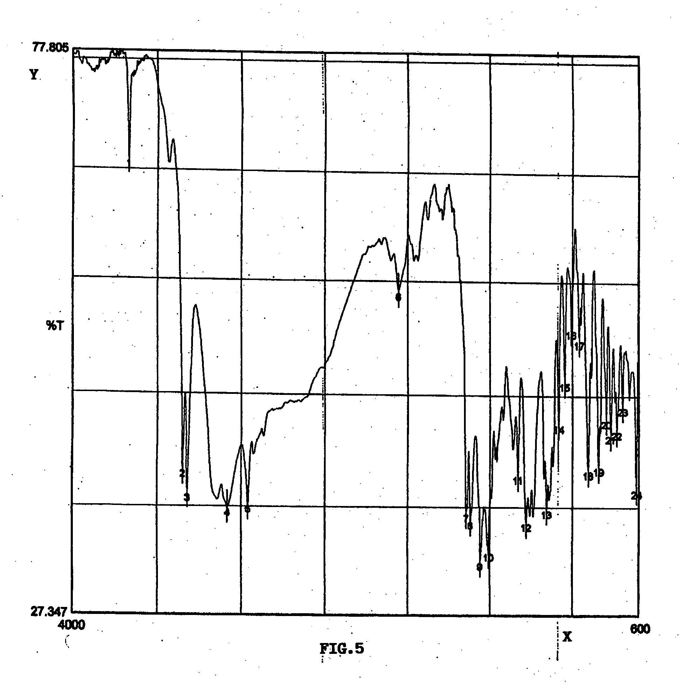

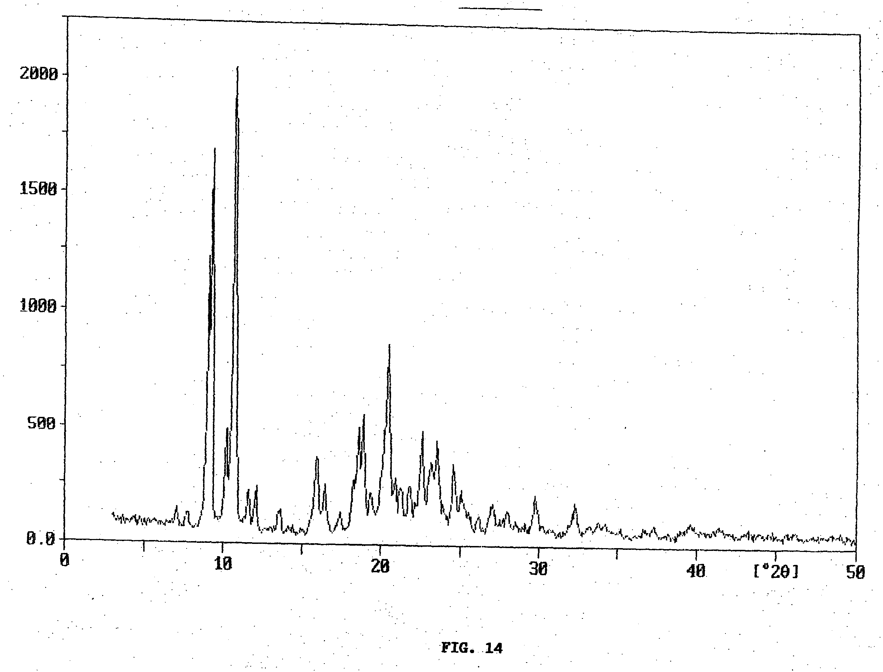

4-(3-methylphenylamino)-3-pyridine-sulphonamide (30 g; 0.114 mol), 1-methyl-2-pyrrolidone (135 cc) are put into a four-necked flask, equipped with mechanical stirrer, thermometer, bubble cooler, and dripping funnel. Keeping the temperature at 20-25°C, there are added by dripping first potassium hydrate 46.3 wt% (13.77 g; 0.114 mol) and then isopropyl isocyanate (10.66 g; 0.125 mol). At the end of the reaction, water (540 cc) is added, the mixture is filtered by gravity, and is then acidified, at 20-25°C, to a pH of 5.5 ± 0.2 with 80% acetic acid (q.s.). The suspension is then stirred for 3 hours at 20-25°C, and is then filtered by suction, thoroughly washing the panel with deionized water. The product is left to dry overnight at 60°C under vacuum, and 36.28 g of torsemide are obtained.
(Yield: 91.4% of the theoretical value)
IR: form II (see Figure 5). DRX: form II (see Figure 14) titre (HClO₄): 99.66%

### EXAMPLE 4

4-(3-methylphenylamino)-3-pyridine-sulphonamide (50 g; 0.189 mol), potassium carbonate (30 g; 0.217 mol), *N,N*-dimethylformamide (200 cc), and deionized water (10 cc) are put into a four-necked flask, equipped with mechanical stirrer, thermometer, bubble cooler, and dripping funnel. The suspension is brought to 70-75°C for 30 min, then is brought to 60-65°C and then, at said temperature, isopropyl isocyanate (18 g; 0.211 mol) is added by dripping. At the end of the reaction, deionized water (800 cc) is added, the solution is filtered by gravity, and is then acidified, at 20-25°C, to a pH of 5.5 ± 0,2 with 80% acetic acid (q.s.).

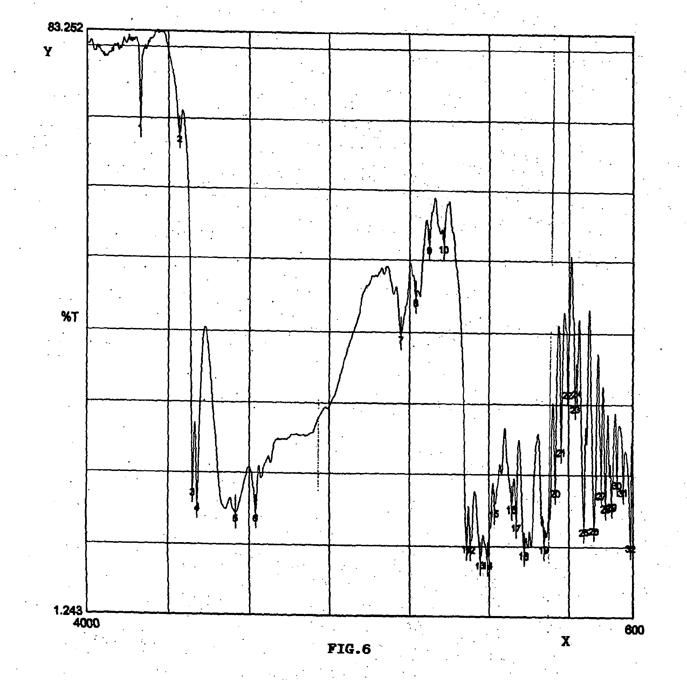

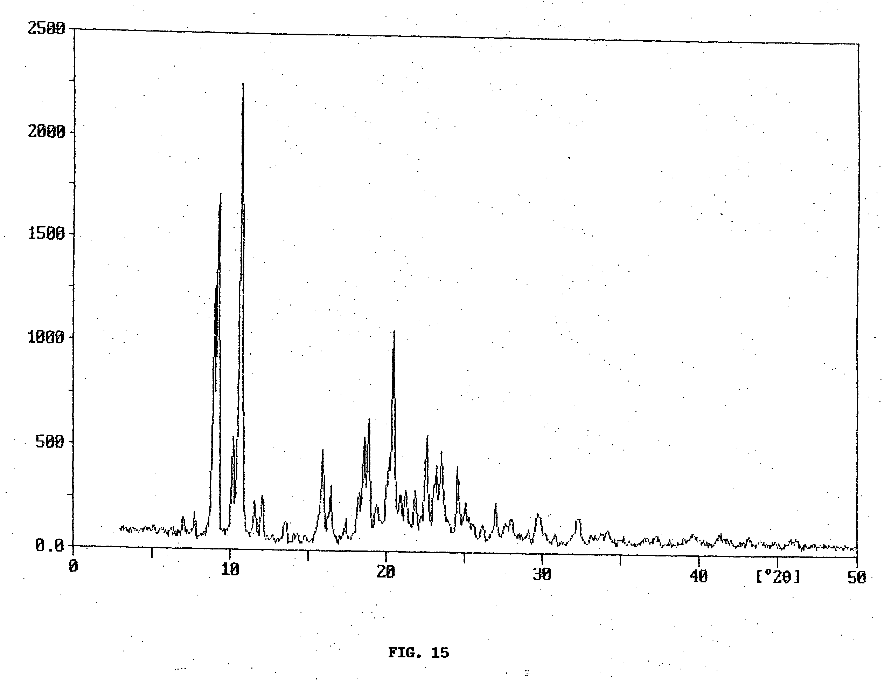

The suspension is then stirred for 30 min at 25°C and is then filtered by suction, thoroughly washing the panel with deionized water. The product is left to dry overnight at 60°C under vacuum, and 61.7 g of torsemide are thus obtained.
(Yield: 93.2% of the theoretical value).
IR: form II (see Figure 6). DRX: form II (see Figure 15) titre (HClO₄): 99.43% titre (HPLC): 99.38%

### EXAMPLE 5

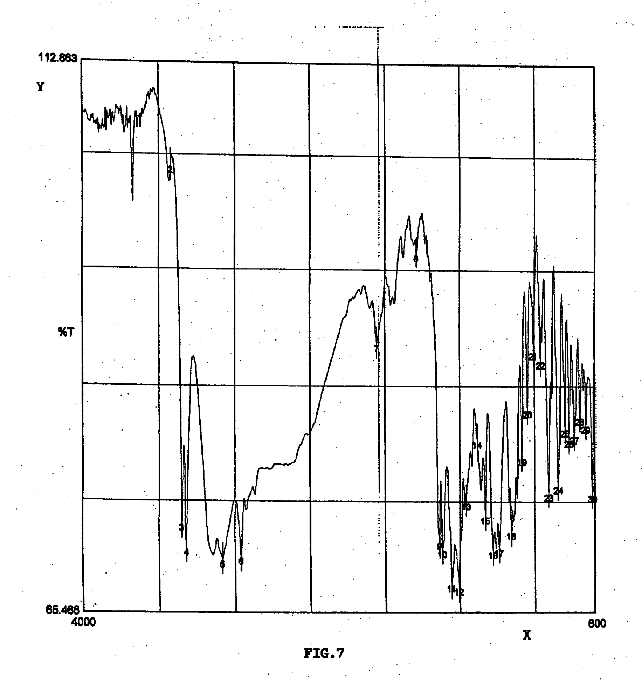

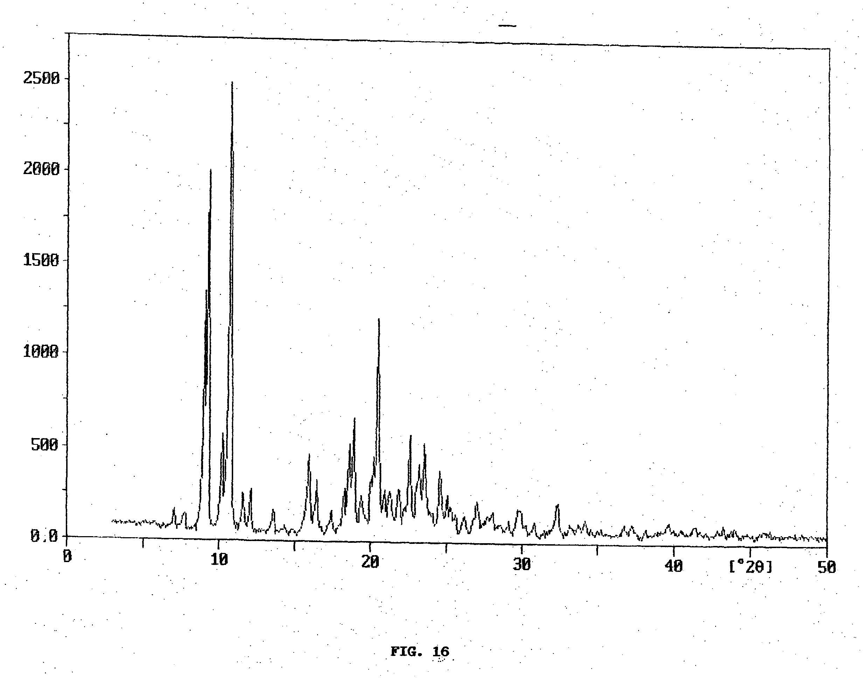

4-(3-methylphenylamino)-3-pyridine-sulphonamide (30 g; 0.114 mol), potassium carbonate (18 g; 0.13 mol), dimethylsulphoxide (120 cc), and water (6 cc) are put into a four-necked flask, equipped with mechanical stirrer, thermometer, bubble cooler, and dripping funnel. The mixture is heated to 70-75°C for 30 min, and is then cooled to 60-65°C, and isopropyl isocyanate (10.68 g; 0.125 mol) is added by dripping at said temperature for 20 min. At the end of the reaction, deionized water (480 cc) is added, the solution is filtered by gravity, and is then acidified, at 20-25°C, to a pH of 5.5 ± 0.2 with 80% acetic acid (q.s.).
The suspension is stirred overnight at 20°C, then filtered by suction, thoroughly washing the panel with deionized water. The product is left to dry overnight a 50°C under vacuum, and 37.1 g of torsemide are thus obtained.
(Yield: 93.5% of the theoretical value).
IR: form II (see Figure 7). DRX: form II (see Figure 16) titre (HPLC): 99.53%

### EXAMPLE 6

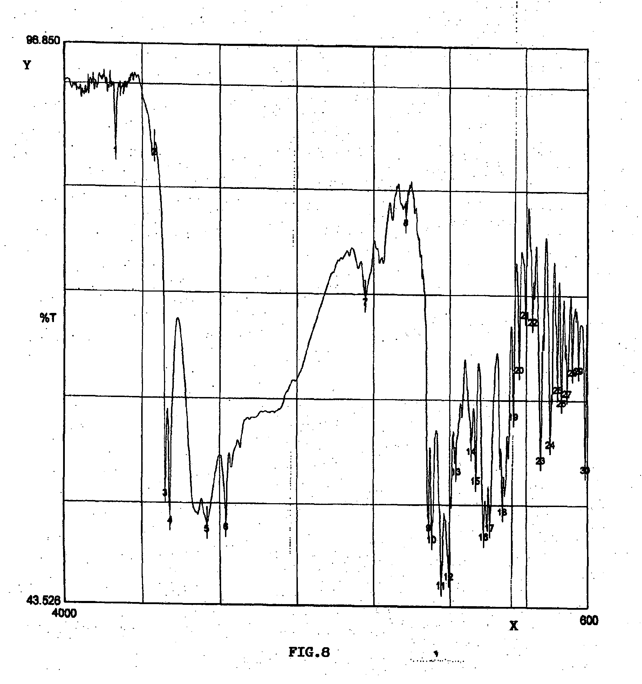

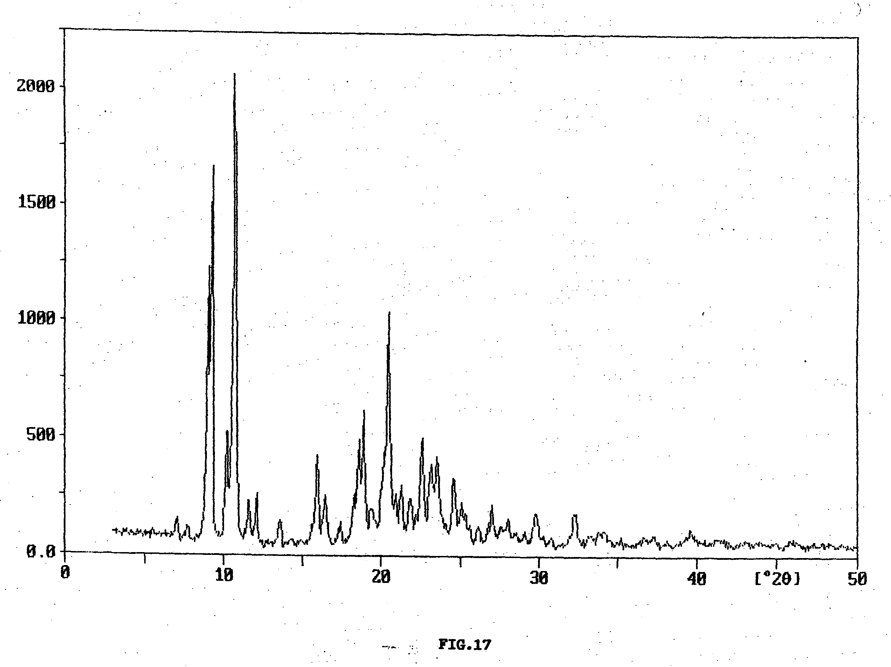

4-(3-methylphenylamino)-3-pyridine-sulphonamide (30 g; 0.114 mol), potassium carbonate (18 g; 0.13 mol), 1.3-dimethyl-2-imidazolidinone (120 cc), and water (6 cc) are put into a four-necked flask, equipped with mechanical stirrer, thermometer, bubble cooler, and dripping funnel. The mixture is heated to 70-75°C for 30 min, and is then cooled to 60-65°C, and isopropyl isocyanate (10.68 g; 0.125 mol) is added by dripping at said temperature in 20 min. At the end of the reaction, deionized water (480 cc) is added, the solution is filtered by gravity, and is then acidified, at 20-25°C, to a pH of 5.5 ± 0.2 with 80% acetic acid (q.s.).
The suspension is then stirred overnight at 20°C, and is then filtered by suction, thoroughly washing the panel with deionized water. The product is left to dry overnight at 50°C under vacuum, and 35.73 g of torsemide are thus obtained. (Yield: 90.2% of the theoretical value).
IR: form II (see Figure 8). DRX : form II (see Figure 17) titre (HClO₄): 99.75%

### EXAMPLE 7

4-(3-methylphenylamino)-3-pyridine-sulphonamide (30 g; 0.114 mol), potassium carbonate (18 g; 0.13 mol), *N,N*-dimethylacetamide (120 cc), and water (6 cc) are put into a four-necked flask, equipped with mechanical stirrer, thermometer, bubble cooler, and dripping funnel. The mixture is heated to 65-70°C for 30 min, and isopropyl isocyanate (10.68 g; 0.125 mol) is then added by dripping at said temperature in 20 min. At the end of the reaction, deionized water (480 cc) is added, the solution is filtered by gravity, and is then acidified, at 20-25°C, to a pH of 5.5 ± 0.2 with 80% acetic acid (q.s.).

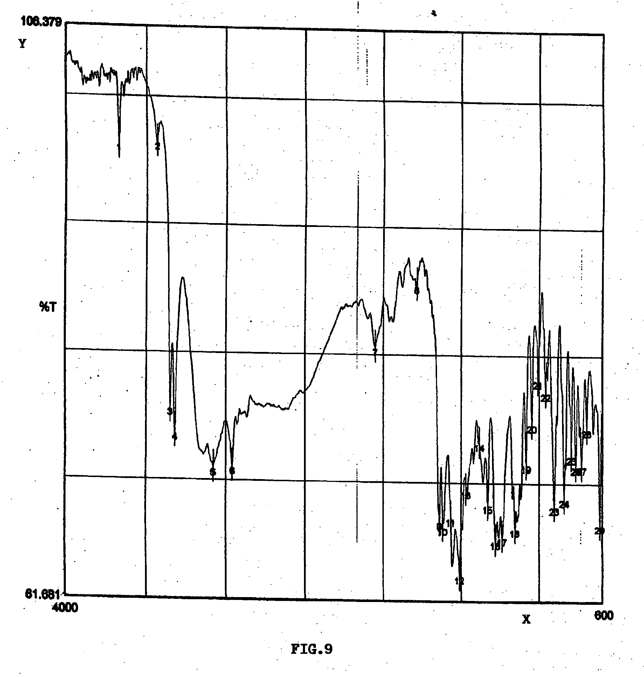

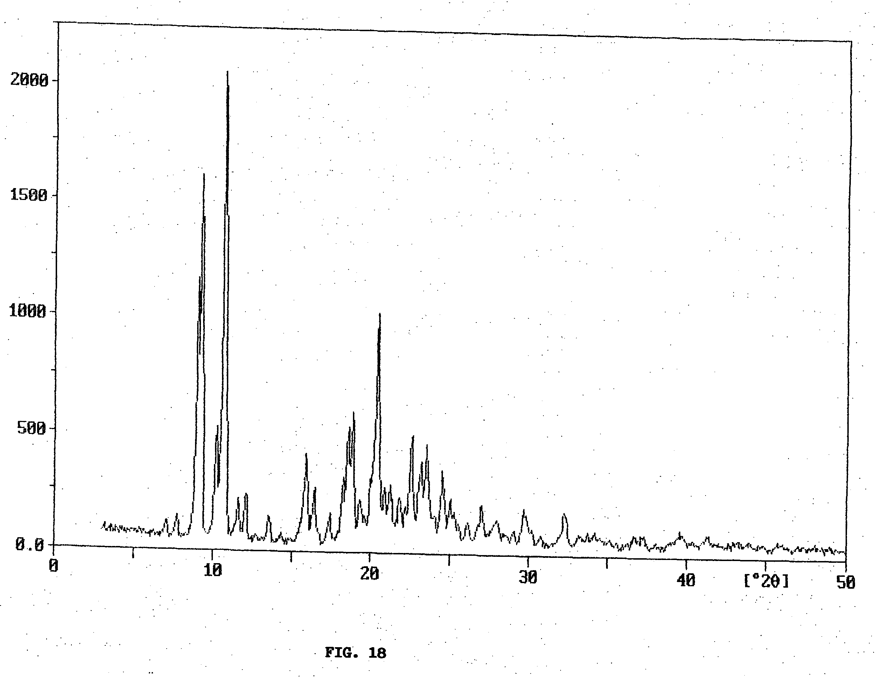

The suspension is then stirred for 3 hours at 20°C, and is then filtered by suction, thoroughly washing the panel with deionized water. The product is left to dry overnight a 50°C, and 37.5 g of torsemide are thus obtained.
(Yield: 94.4% of the theoretical value).
IR: form II (see Figure 9). DRX: form II (see Figure 18)
titre (HClO₄): 99.67%

### EXAMPLE 8.

4-(3-methylphenylamino)-3-pyridine-sulphonamide (30 g; 0.114 mol), acetone (135 cc), and sodium hydrate 30% (15.19 g; 0.114 mol) are put into a four-necked flask, equipped with mechanical stirrer, thermometer, bubble cooler, and dripping funnel. To the solution thus obtained, keeping the temperature at 20-30°C, isopropyl isocyanate (10.68 g; 0.125 mol) is added by dripping. At the end of the reaction, deionized water (540 cc) is added, the solution is filtered by gravity, and is then acidified, at 20-25°C, to a pH of 5.5 ± 0.2 with 80% acetic acid (q.s.).

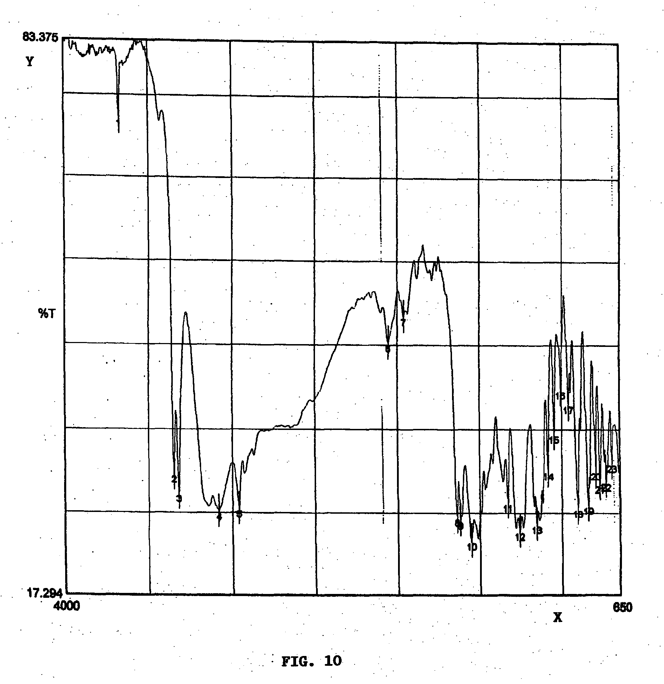

The suspension is stirred per 15 min at 20-25°C, and is then filtered by suction, thoroughly washing the panel with deionized water. The product is left to dry overnight at 65°C under vacuum, and 38.1 g of torsemide are thus obtained.
(Yield: 96% of the theoretical value).
IR: form II (see Figure 10). titre (HClO₄): 99.63%

### EXAMPLE 9

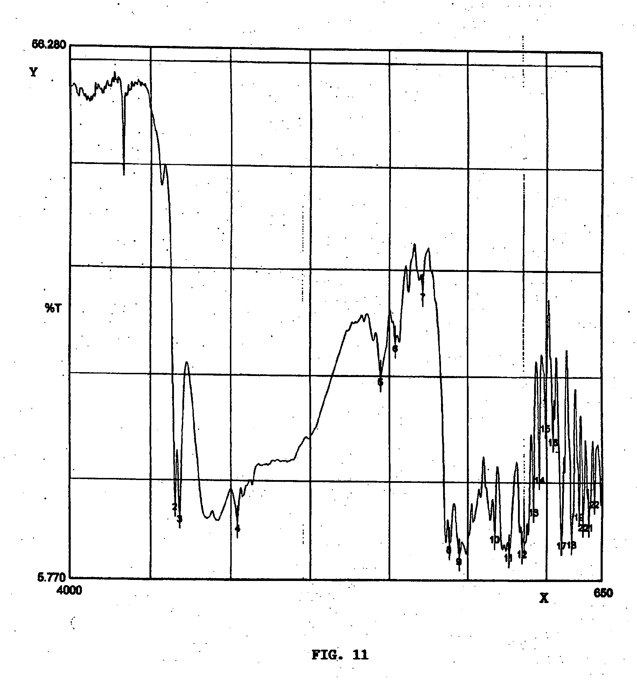

4-(3-methylphenylamino)-3-pyridine-sulphonamide (30 g; 0.114 mol), water (3.3 cc), dimethylformamide (5 cc), acetone (120 cc), and potassium carbonate (18 g; 0.13 mol) are put into a four-necked flask, equipped with mechanical stirrer, thermometer, bubble cooler, and dripping funnel. The mixture is heated to 55-60°C for 30 min, then isopropyl isocyanate (10.17 g; 0.119 mol) is added by dripping, maintaining said temperature. Upon completion of the reaction, first acetone (30cc) is added to fluidify the mixture even more, then deionized water (600 cc) is added, the solution is filtered by gravity, and is then acidified, at 20-25°C, to a pH of 5.5 ± 0.2 with 80% acetic acid (q.s.). The suspension is stirred for 15 min at 20-25°C, and is then filtered by suction, thoroughly washing the panel with deionized water. The product is left to dry overnight at 70°C under vacuum, and 38 g of torsemide are thus obtained.
(Yield: 95.7% of the theoretical value).
IR: form II (see Figure 11). titre (HClO₄): 99.99%

### EXAMPLE 10

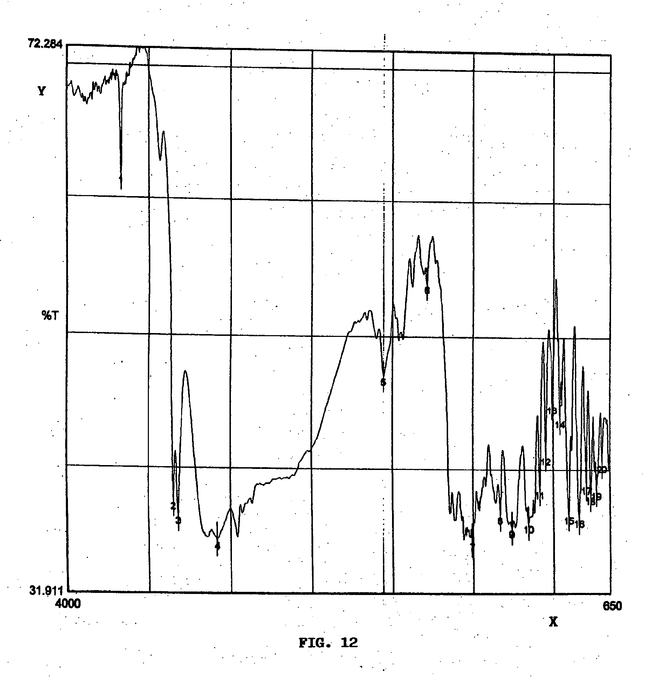

4-(3-methylphenylamino)-3-pyridine-sulphonamide (30 g; 0.114 mol), dimethylformamide (120 cc), and potassium carbonate (18 g; 0.13 mol) are put into a four-necked flask, equipped with mechanical stirrer, thermometer, bubble cooler, and dripping funnel. The mixture is heated to 60-65°C for 30 min, then, maintaining the internal temperature at 55-65°C, isopropyl isocyanate (11.72 g; 0.138 mol) is added by dripping. At the end of the reaction, deionized water (480 cc) is added, the solution is filtered by gravity, and is then acidified, at 20-25°C, to a pH of 5.5 ± 0.2 with 80% acetic acid (q.s.). The suspension is stirred for one hour at 20-25°C, and is then filtered by suction, thoroughly washing the panel with deionized water. The product is left to dry overnight at 70°C under vacuum, and 37.7 g of torsemide are thus obtained.
(Yield: 94.9% of the theoretical value).
IR: form II (see Figure 12) titre (HClO₄): 99.45% titre (HPLC): 99.86%.

### Description of the analytic determinations:

The FT-IR spectra of torsemide were recorded with a 300E Jasco apparatus at a scanning speed of 2.0 mm/s with an amplification of 16 in the spectral range between 4000 and 600 cm⁻¹ and with a resolution of 4.00 cm⁻¹ using the diffused-reflectance technique (KBr).

The x-ray diffractograms of the powders of torsemide were recorded with a Philips PW 1032/1710 diffractometer with copper anode (λ=1.54 Angstrom, alpha1 = 1.54060 Angström; alpha2 = 1.54439 Angstrom; alpha2/alpha1 = 0.500), with divergence slit of one half of a degree and a receiving slit of 0.1 degrees, at a generator voltage of 40 kV and a current of 30 mA. Scanning was carried out in continuous mode between 3 and 60 degrees two-theta, with a measurement step of 0.020 degrees two-theta and with a time per step of 1.250 s.

The HClO₄ potenziometric titre of torsemide was measured with a "Metrohm 785 DMP Titrino" or else "716 DMS Titrino" or else "665 Dosimat/682 Titroprocessor" apparatus, with the use of a "Metrohm 6.0232.100 glass-combined" electrode, using 0.1N HClO₄ as titrant.

The HPLC titre of torsemide (established according to US Pharmacopoeia, in force from January, 2002, which requires a HPLC titre equal to or higher than 98.0%) was measured using an Agilent 1100 apparatus, provided with a 288-nm detector, and a 150 x 4.6-mm column charged with 7-µm particles of "Hypersil ODS".

## Claims

1. A process for the synthesis of torsemide, comprising the following steps:
- providing a reactive mixture comprising 4-(3-methylphenylamino)-3-pyridine-sulphonamide, an aprotic solvent mixable with water or a mixture of aprotic solvents mixable with water and an inorganic base;
- adding, by dripping, to the reactive mixture, isopropyl isocyanate in an amount substantially equimolar to the 4-(3-methylphenylamino)-3-pyridine-sulphonamide included in the reactive mixture, keeping the temperature between 0°C and 70°C;
- optionally adding water upon completion of the reaction;
- removing, by filtration, any insoluble substances that may have been formed; and
- precipitating by acidification the torsemide that has formed.

2. The process according to Claim 1, wherein the reactive mixture also comprises water as a further component.

3. The process according to Claim 1 or 2, wherein the aprotic solvent mixable with water is chosen from the group consisting of: acetone, acetonitrile, diglyme, *N,N*-dimethylformamide, dimethylsulphoxide, *N*-methyl-2-pyrrolidone, dimethylacetamide, and 1,3-dimethyl-2-imidazolidinone.

4. The process according to Claim 3, wherein the aprotic solvent mixable with water is chosen from the group consisting of: acetone, diglyme, *N,N*-dimethylformamide, dimethylsulphoxide, *N*-methyl-2-pyrrolidone, dimethylacetamide and 1,3-dimethyl-2-imidazolidinone.

5. The process according to any one of the preceding claims, in which the inorganic base is chosen from the group consisting of: potassium hydrate, sodium hydrate, sodium carbonate and potassium carbonate.

6. The process according to Claim 5; wherein the inorganic base is chosen from the group consisting of potassium hydrate and potassium carbonate.

7. The process according to Claim 6, wherein the inorganic base is potassium carbonate.

8. The process according to any one of the preceding claims, wherein, in the reactive mixture, the 4-(3-methylphenylamino)-3-pyridine-sulphonamide is present at 12-22 wt%, water is present at 0-50 wt%, the inorganic base is present at 3-14 wt%, and the aprotic solvent mixable with water is present at 40-80 wt%.

9. The process according to any one of the preceding claims, wherein upon completion of the reaction, the reaction mixture is diluted with 0-4 parts by weight of water, referred to the weight of the reactive mixture.

10. The process according to any one of the preceding claims, wherein the torsemide which has formed is precipitated by adding an acid to yield a pH of 5.5 ± 0.2 at a temperature of 20-25°C.

11. The process according to Claim 10, wherein the acid added is acetic acid.

12. The process for the production of pharmaceutical preparations that comprise torsemide, comprising the synthesis of torsemide according to any one of the preceding claims and its subsequent mixing to suitable excipients.

13. The process according to Claim 12, in particular for the preparation of solid pharmaceutical forms, comprising, prior to mixing with the excipients, a step in which the polymorphous form of the starting torsemide is micronized or converted into another polymorphous form or else into an amorphous form.

14. The process for production of pharmaceutical preparations that comprise pure and stable form II torsemide, comprising the synthesis of torsemide according to Claim 4 and its subsequent mixing with suitable excipients.
